# EUROPEAN PATENT APPLICATION

(11) **EP 4 606 425 A1**
(43) Date of publication of application: **27.08.2025**
(21) Application number: 23881748.0
(22) Date of filing: 20.10.2023
(51) Int. Cl.: A61N 5/10

(54) **BORON NEUTRON CAPTURE THERAPY SYSTEM AND WORKING METHOD THEREFOR**

(30) Priority: 24.10.2022 CN 202211302487
(71) Applicant: Neuboron Therapy System Ltd., Xiamen, Fujian 361026 (CN)
(72) Inventor: LIU, Yuanhao, Nanjing, Jiangsu 211112 (CN); CHEN, Jiang, Nanjing, Jiangsu 211112 (CN)
(74) Representative: Gunzelmann, Rainer
(86) International application number: PCT/CN2023/125637
(87) International publication number: WO 2024/088170

(57) **Abstract**

A boron neutron capture therapy system and a working method therefor. The boron neutron capture therapy system comprises: a boron concentration acquisition module, which is used for acquiring blood boron concentration data of an irradiated object; a neutron beam irradiation module, which is used for generating a neutron beam; a therapy plan module, which is used for generating a therapy plan; a placement module, which is at least used for calculating a placement position of the irradiated object; a carrying module, which is used for moving the irradiated object to the placement position; and a data management module, which is used for storing information data generated by at least one of the modules and/or performing information interaction with at least one of the modules. Accordingly, resource waste and radiation damage which are caused by unnecessary startup are avoided, the operation efficiency of each device is improved, the process of data processing is simplified, the degree of automation of the system is improved, and the operation complexity of the system is reduced.

## Description

### TECHNICAL FIELD

The present disclosure relates to the technical field of radiotherapy, and in particular to a boron neutron capture therapy (BNCT) system and a working method thereof.

### BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

**In** order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, BNCT in neutron capture therapy serves as a better alternative to treat the cancers.

The BNCT has been applied increasingly in recent years as an effective method to treat the cancers. However, from clinical practices, the whole treatment process includes many steps and correspondingly involves many devices. Specifically, the treatment steps include image information acquisition, treatment scheme formulation, simulated setup, setup before irradiation, the irradiation, etc. The involved devices include an image acquisition device, a data management device, a control system, a setup system, etc. Different steps correspond to different devices. During treatment, information acquired by these devices is to be integrated to control the treatment progress. Usually, these devices are provided by different suppliers, and each of them works as an independent unit. They cannot realize information interaction, and cannot cooperate with each other. Moreover, some devices cannot keep an operating state throughout the whole treatment process, and information interaction of the devices in the treatment is problematic.

### SUMMARY

In view of the above-mentioned technical problems, the present disclosure provides a BNCT system and a working method thereof, to facilitate the information interaction and improve the degree of automation of the system.

According to an aspect, the present disclosure provides a BNCT system, including:
a boron concentration acquisition module configured to acquire blood boron concentration data of an irradiated body;
a neutron beam irradiation module configured to generate a neutron beam;
a treatment planning module configured to generate a treatment plan;
a setup module at least configured to calculate a setup position of the irradiated body;
a placement module configured to move the irradiated body to the setup position; and
a data management module configured to store information data generated by at least one of the boron concentration acquisition module, the neutron beam irradiation module, the treatment planning module, the setup module, and the placement module, and/or perform information interaction with at least one of the boron concentration acquisition module, the neutron beam irradiation module, the treatment planning module, the setup module, and the placement module.

Further, the BNCT system further includes an image acquisition module configured to acquire medical image data of the irradiated body; the treatment planning module is configured to generate the treatment plan based on the medical image data; and the treatment planning module is configured to perform information data interaction with the data management module and store the treatment plan to the data management module.

Further, the treatment planning module is configured to acquire a voxel prosthesis tissue model based on the medical image data of the irradiated body, and perform simulation based on the voxel prosthesis tissue model to generate the treatment plan.

Further, the setup module is configured to acquire the treatment plan from the data management module, and perform calculation based on the treatment plan to obtain a preset setup position.

Further, the setup module is configured to acquire the treatment plan from the data management module, and perform calculation based on the treatment plan to obtain a simulated setup position.

Further, the setup module is configured to perform calculation based on the simulated setup position to obtain a setup motion parameter; and the placement module is configured to move the irradiated body based on the setup motion parameter to a treatment setup position.

Further, after the irradiated body is moved to the treatment setup position, the placement module is configured to generate setup in-place information and transmit the setup in-place information to the data management module.

Further, the BNCT system further includes an irradiation control module; and the irradiation control module is configured to acquire the setup in-place information and the treatment plan from the data management module, and control the neutron beam irradiation module based on the treatment plan to generate a neutron beam.

According to another aspect, the present disclosure provides a working method of a BNCT system, including the following steps:
generating, by a treatment planning module, a treatment plan based on medical image data;
storing the treatment plan to a data management module;
acquiring, by a setup module, the treatment plan from the data management module, and calculating a setup position of an irradiated body based on the treatment plan;
moving, by a placement module, the irradiated body to the setup position; and
acquiring, by an irradiation control module, the treatment plan from the data management module, and controlling a neutron beam irradiation module based on the treatment plan to generate a neutron beam.

Further, the treatment planning module is configured to define a tissue boron concentration in the medical image data of the irradiated body, acquire a voxel prosthesis tissue model having a tissue type and the tissue boron concentration, and perform simulation based on the voxel prosthesis tissue model to generate the treatment plan.

Further, the setup module is configured to acquire the treatment plan from the data management module, acquire a preset setup position based on the treatment plan, evaluate whether the preset setup position meets a preset condition, update the treatment plan if the preset setup position does not meet the preset condition, and store an updated treatment plan to the data management module.

Further, the setup module is configured to acquire the treatment plan from the data management module, acquire a simulated setup position based on the treatment plan, and further perform calculation based on the simulated setup position to obtain a setup motion parameter; and the setup module is configured to control the placement module based on the setup motion parameter to move the irradiated body to a treatment setup position.

Further, the setup module is configured to acquire the treatment plan from the data management module, acquire a simulated setup position based on the treatment plan, perform calculation based on the simulated setup position to obtain a setup motion parameter, and store the setup motion parameter to the data management module; and the placement module is configured to acquire the setup motion parameter from the data management module, and move the irradiated body based on the setup motion parameter to a treatment setup position.

According to the present disclosure, the BNCT system includes the data management module configured to store information generated by at least one of the boron concentration acquisition module, the treatment planning module, the neutron beam irradiation module, the setup module, the placement module, and the simulation placement module. In work of the BNCT system, relevant data can be transmitted to the data management module by at least one of remaining modules, and desired data can be acquired from the data management module to perform corresponding calculation and/or control. Hence, the associated module can be turned off or works in a next round after completing the corresponding work. This prevents the resource waste and the radiation damage caused by unnecessary startup, and improves the operation efficiency of each device. In addition, the data management module receives and stores the information data generated by at least one of the boron concentration acquisition module, the neutron beam irradiation module, the setup module, the treatment planning module, the placement module, and the irradiation control module 9, and/or perform information interaction with at least one of these modules. This simplifies the data processing procedure, improves the degree of automation of the BNCT system, and reduces the operation complexity.

### BRIEF DESCRIPTION OF THE DRAWINGS

FIG. 1 is a structural view of a BNCT system according to the present disclosure;
FIG. 2 is a structural view of a layout of a BNCT system according to the present disclosure;
FIG. 3 is a schematic view of a beam shaper according to the present disclosure; and
FIG. 4 is a schematic view of a placement module and a positioning module in an irradiation room of a BNCT system according to the present disclosure.

In the figures: 1-image acquisition module, 2-boron concentration acquisition module, 3-neutron beam irradiation module, 31-neutron generation device, 311-accelerator, 312-target, 32-beam shaper, 321-moderator, 322-reflector, 323-thermal neutron absorber, 324-radiation shield, 325-beam outlet, 33-collimator, 4-setup module, 5-treatment planning module, 6-data management module, 7-placement module, 71-placement table, 72-mechanical arm, 73-drive member, 8-simulation placement module, 82-simulation placement table, 81-simulation beam outlet, 9-irradiation control module, 20-irradiation room, 21-first positioning device, 30-preparation room, 31-second positioning device, and S-irradiated body.

### DETAILED DESCRIPTION OF THE EMBODIMENTS

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

Preferably, embodiments of the present disclosure are further described below in detail with a neutron capture therapy system, and particularly with a BNCT system.

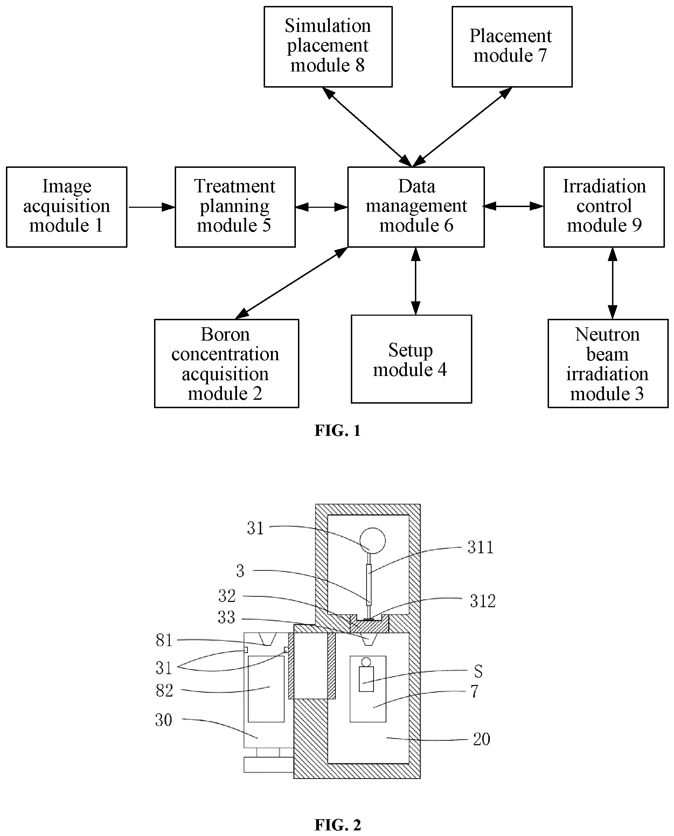

As shown in FIG. 1, a BNCT system in the embodiment includes an image acquisition module 1, a boron concentration acquisition module 2, a neutron beam irradiation module 3, a setup module 4, a treatment planning module 5, a data management module 6, a placement module 7, and an irradiation control module 9. The image acquisition module 1 is configured to acquire a lesion-containing three-dimensional (3D) medical image of an irradiated body S. The boron concentration acquisition module 2 is configured to detect a boron concentration in blood of the irradiated body S. The neutron beam irradiation module 3 is configured to generate a neutron beam for treatment. The treatment planning module 5 is configured to perform dose simulation and calculation according to the 3D medical image of the irradiated body S and generate a treatment plan. The treatment plan includes an irradiation position, an irradiation direction, irradiation time, etc. The setup module 4 is at least configured to calculate a setup position of the irradiated body S according to the treatment plan generated by the treatment planning module 5. The placement module 7 is configured to move the irradiated body S to the setup position. The irradiation control module 9 is configured to control a whole treatment progress according to the treatment plan. The data management module 6 is at least configured to receive and store information data generated by at least one of the boron concentration acquisition module 2, the neutron beam irradiation module 3, the setup module 4, the treatment planning module 5, the placement module 7, and the irradiation control module 9, and/or perform information interaction with at least one of the boron concentration acquisition module 2, the neutron beam irradiation module 3, the setup module 4, the treatment planning module 5, the placement module 7, and the irradiation control module 9.

The main principle of the BNCT is as follows: A boron (^{B}-10)-containing drug taken by or injected into the irradiated body S is gathered to the tumor cells selectively. With a large capture cross section to thermal neutrons, and through ^{M}B(n,α)⁷ Li neutron capture and a nuclear fission reaction, the boron(^{B}-10)-containing drug generates ⁴He and ⁷Li heavy charged particles. Two heavy charged particles have an average energy of about 2.33 MeV, and have characteristics of high linear energy transfer (LET) and the short range. A total range of the two particles is approximately equivalent to a cell size, such that the radiation damage to an organism is limited to a cell level, and a purpose of locally killing the tumor cells on premise of no serious damage to normal tissues can be achieved.

The device for acquiring the 3D medical image may be an imaging device such as a CT device, an MRI device, a PET device and an ultrasound device. In the present disclosure, preferably, the image acquisition module 1 of the CT device is used to acquire medical image data of the irradiated body S through CT. The medical image data of the irradiated body S includes a coordinate matrix and a CT value matrix of a medical image voxel model of a to-be-irradiated portion (a lesion, namely the tumor cells) in a medical image coordinate system. The image acquisition module 1 transmits the medical image data to the data management module 6.

The boron concentration may be detected by an inductively coupled plasma atomic emission spectroscopy (ICP-AES), a high-resolution alpha-particle autoradiography, a charged ion spectroscopy, a neutron capture camera, a nuclear magnetic resonance (NMR) and an MRI, a PET, an instantaneous gamma-ray spectrometer, etc. The device involved in the above detection methods is called the boron concentration acquisition module 2. After the boron (^{B}-10)-containing drug is taken by or injected into the irradiated body S for a period of time, the boron concentration acquisition module 2 acquires a boron concentration of the irradiated body S. The boron concentration acquisition module 2 transmits a detected boron concentration to the data management module 6.

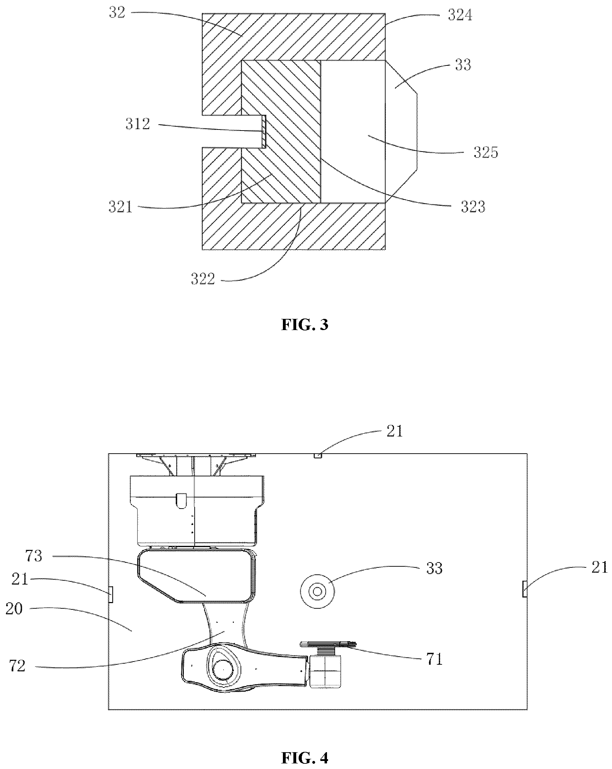

Referring to FIG. 2 and FIG. 3, in the embodiment of the present disclosure, the neutron beam irradiation module 3 includes a neutron generation device 31, a beam shaper 32, and a collimator 33. The neutron generation device 31 includes an accelerator 311 and a target 312. The accelerator 311 is configured to accelerate charged particles (such as protons and deuterons) to generate a charged particle line such as a proton line. The charged particle line is irradiated onto the target 312 and generates a neutron line (a neutron beam) under an action of the target 312. The target 312 is preferably a metal target 312. An appropriate nuclear reaction is selected according to characteristics such as a desired neutron yield and energy, available energies of the accelerated charged particles, a current, physical and chemical properties of the metal target 312, or the like. Nuclear reactions commonly discussed include ⁷Li(p,n) ⁷Be and ⁹Be(p,n) ⁹B, both of which are endothermic reactions. In the embodiment of the present disclosure, the target 312 is made of lithium. However, as is known to those skilled in the art, the target 312 may also be made of a metal material other than the lithium and beryllium, such as tantalum (Ta) or tungsten (W). The target 312 may be a circular plate, and may also be other solid shapes, and may also be made of a liquid (liquid metal). The accelerator 311 may be a linear accelerator, a cyclotron, a synchrotron and a synchrocyclotron. In other implementations, the neutron generation device 31 may be a nuclear reactor without the accelerator 311 and the target 312.

No matter whether a neutron source in the BNCT comes from the nuclear reactor or the nuclear reaction between the accelerated charged particles and the target 312, a mixed radiation field is generated as a matter of fact, that is, the generated beam includes neutrons and photons having energies from low to high. As for the BNCT on deep tumors, except the epithermal neutrons, the higher the content of remaining radioactive rays, the greater the proportion causing non-selective dose deposition in the normal tissue. Therefore, the content of radioactive rays causing the unnecessary dose deposition should be reduced as much as possible. The beam shaper 32 can adjust quality of the neutron beam generated by the neutron generation device 31, thereby reducing the unnecessary dose deposition. The collimator 33 is configured to gather the neutron beam, such that the neutron beam has a high targeting ability in the treatment.

The beam shaper 32 includes a moderator 321, a reflector 322, a thermal neutron absorber 323, a radiation shield 324, and a beam outlet 325. The moderator 321 can adjust an energy range (>40 keV) of fast neutrons generated by the neutron generation device 31 to an energy range (0.5 eV to 40 keV) of epithermal neutrons, and reduce a content of thermal neutrons (<0.5 eV) as much as possible. The moderator 321 is made of a material having a large action section with the fast neutrons but a small action section with the epithermal neutrons. As a preferred embodiment, the moderator 321 is made of at least one of D₂O, AlF₃, Fluental_{TM}, CaF₂, Li₂CO₃, MgF₂, and Al₂O₃. The reflector 322 surrounds the moderator 321, and reflects neutrons diffused through the moderator 321 back to the neutron beam, thereby improving the utilization rate of the neutrons. The moderator is made of a material with a strong neutron reflectivity. As a preferred embodiment, the reflector 322 is made of at least one of Pb or Ni. On a transmission path of the neutron beam, the thermal neutron absorber 323 is provided behind the moderator 321, configured to absorb thermal neutrons passing through the moderator 321 to reduce the content of the thermal neutrons in the neutron beam, and made of a material having a large action section with the thermal neutrons. As a preferred embodiment, the thermal neutron absorber 323 is made of Li⁻⁶. In other embodiments, because of the Li⁻⁶ in the material of the moderator 321, the thermal neutron absorber 323 may not be provided individually, but the moderator 321 serves as the thermal neutron absorber 323. The radiation shield 324 is configured to shield neutrons and photons leaked from a portion other than the beam outlet 325. A material of the radiation shield 324 includes at least one of a photon shielding material and a neutron shielding material. As a preferred embodiment, the material of the radiation shield 324 includes lead (Pb) as the photon shielding material and polyethylene (PE) as the neutron shielding material.

The collimator 33 is provided behind the beam outlet 325. An epithermal neutron beam from the collimator 33 is irradiated onto the irradiated body S. After passing through a superficial normal tissue of the irradiated body S, the epithermal neutron beam is moderated as thermal neutrons to reach the tumor cells for treatment.

It may be understood that the beam shaper 32 may also be other structures, provided that the epithermal neutron beam can be obtained to meet the treatment requirement. In the present disclosure, the collimator 33 may also not be provided, and the beam from the beam outlet 325 of the beam shaper 32 is directly irradiated onto the irradiated body S. For ease of description, when the collimator 33 is provided, an outlet of the collimator 33 may also be understood as the beam outlet 325.

The treatment planning module is configured to acquire the image data from the image acquisition module 1, perform the dose simulation and calculation, generate the treatment plan, and transmit the treatment plan to the data management module 6. Specifically, the treatment planning module 5 is configured to convert the CT value matrix in the medical image data into a tissue type information matrix to obtain a voxel prosthesis tissue model having a tissue type. Further, the treatment planning module 5 may further be configured to define a tissue boron concentration in the medical image data of the irradiated body S. The treatment planning module 5 may be configured to acquire a voxel prosthesis tissue model having the tissue type and the tissue boron concentration, and perform the simulation and the calculation based on the voxel prosthesis tissue model to generate the treatment plan. It may be understood that the data processing procedure for converting the CT value matrix in the medical image data into the tissue type information matrix and defining the tissue boron concentration in the medical image data of the irradiated body S to obtain the voxel prosthesis tissue model having the tissue type and the tissue boron concentration may also be performed in the data management module 6.

Referring to FIG. 2 and FIG. 4, the placement module 7 includes a placement table 71 configured to place the irradiated body S, a mechanical arm 72 connected to the placement table 71 and configured to drive the placement table 71 to move, and a drive member 73 configured to drive the mechanical arm 72 to move.

The whole BNCT system is accommodated in a concrete building. Specifically, the BNCT system further includes a preparation room 30 and an irradiation room 20. The placement table 71 is configured to place the irradiated body S to receive neutron beam irradiation in the irradiation room 20 for treatment. One or more irradiation rooms 20 correspond to one preparation room 30. The preparation room 30 is configured to perform preparatory work before the irradiating treatment, such as pre-setup of the irradiated body S, and injection of the boron-containing drug. With the pre-setup in the preparation room 30, the working time for setting up the irradiated body S before the irradiating treatment in the irradiation room 20 can be saved. While the preparatory work is performed in the preparation room 30, another irradiated body S can receive the irradiating treatment in the irradiation room 20. This increases the utilization rate of the device. Further, the BNCT system further includes a simulation placement module 8 provided in the preparation room 30. The simulation placement module 8 includes a simulation placement table 82, a simulation mechanical arm (not shown), a simulation drive portion (not shown), and a simulation beam outlet 81.

The beam outlet 325 of the neutron beam irradiation module 3 is at least partially formed in the irradiation room 20. The placement table 71, the mechanical arm 72, and the drive portion are provided in the irradiation room 20. The simulation beam outlet 81, the simulation placement table 82, the simulation mechanical arm, and the simulation drive portion are provided in the preparation room 30. Structures and relative positions of the simulation beam outlet 81, the simulation placement table 82, and the simulation mechanical arm are respectively the same as structures and relative positions of the beam outlet 325, the placement table 71, and the mechanical arm 72 in the irradiation room 20.

In the embodiment of the present disclosure, the placement table 71 and the mechanical arm 72, as well as the simulation placement table 82 and the simulation mechanical arm, are connected together stably in a detachable manner. Upon simulated setup of the irradiated body S on the simulation placement table 82 in the preparation room 30, while the relative position relationship between the irradiated body S and the simulation placement table 82 is unchanged, the simulation placement table 82 is detached from the simulation mechanical arm. Then, the simulation placement table 82 tied with the irradiated body S is transported to the irradiation room 20 through a device such as a trolley and provided on the mechanical arm 72 of the setup module 4. This can restore the relative position relationship between the irradiated body S and the placement table 71 in pre-setup in the irradiation room 20 maximally and simply, and saves the preparation time in the surgery. In addition, the placement table 71 and the simulation placement table 82 are a same component, which saves the device cost, and can reduce a number of times that the irradiated body S is tied to the placement table 71.

Referring also to FIG. 2 and FIG. 4, in order to improve the setup accuracy, a first positioning device 21 and a second positioning device 31 are respectively provided in the irradiation room 20 and the preparation room 30. In the embodiment of the present disclosure, the first positioning device 21 and the second positioning device 31 each are composed of three laser emitters. Specifically, the laser emitters are respectively provided at three sides of each of the beam outlet 325 and the simulation beam outlet 81. Three laser beams generated by the three laser emitters intersect at a point. Further, the beam outlet 325 and the simulation beam outlet 81 each are provided with an auxiliary laser positioning device. A laser line of the auxiliary laser positioning device coincides with a central axis of each of the beam outlet 325 and the simulation beam outlet 81. An intersection point for three laser beams of the first positioning device 21 and an intersection point for three laser beams of the second positioning device 31 respectively fall onto the central axis of the beam outlet 325 and the central axis of the simulation beam outlet 81. In the embodiment of the present disclosure, the three laser emitters are respectively provided at a left side, a right side, and a top side of the beam outlet 325, and specifically may be provided on a left wall, a right wall, and a top ceiling of the beam outlet 325. Certainly, positions of the three laser emitters are not limited thereto.

The present disclosure further provides a working method of a BNCT system, including the following steps:
A treatment plan is generated:
A treatment planning module 5 performs dose simulation and calculation based on medical image data, and generates the treatment plan. Specifically, an image acquisition module 1 transmits the medical image data of a to-be-irradiated body 3 to the treatment planning module 5. The treatment planning module 5 performs the simulation and the calculation based on the medical image data to generate the treatment plan. The treatment plan includes an irradiation position, an irradiation direction, irradiation time, and the like in at least one preset boron concentration. Certainly, in other preferred implementations, the treatment plan includes an irradiation position, an irradiation direction, irradiation time, and the like in a plurality of different preset boron concentrations.

More specifically, before treatment, the image acquisition module 1 is used to scan an image of the irradiated body S to acquire the 3D medical image data at least including a lesion for the irradiated body S. The image acquisition module 1 transmits the medical image data of the irradiated body S to the treatment planning module 5. Based on the medical image data, the treatment planning module 5 establishes a voxel prosthesis tissue model having a tissue type and a tissue boron concentration, and simulates collision trajectories and energy distributions of nuclear particles in an internal 3D space through a Monte Carlo simulation program when the irradiated body S is irradiated by a neutron beam. Through sampling at different irradiation angles, a dose distribution of the 3D voxel prosthesis tissue model at the different irradiation angles and different irradiation time is simulated and calculated. In combination with the dose distribution as well as a dose index (such as a prescribed dose), and a dose limit (such as an average dose and a maximum dose), the treatment plan is generated. A neutron beam model established based on beam source data of the neutron irradiation module 3 is provided in the treatment planning module 5.

In other optional implementations, before treatment, a boron-containing drug is taken by or injected into the irradiated body S. After drug deposition for predetermined time, the image acquisition module 1 is used to scan an image of the irradiated body S to acquire the 3D medical image data at least including a lesion for the irradiated body S. A boron concentration acquisition module 2 is used to acquire a boron concentration of the irradiated body S. The image acquisition module 1 transmits the medical image data of the irradiated body S to the treatment planning module 5. The boron concentration acquisition module 2 transmits boron concentration data to a data management module 6. The treatment planning module 5 acquires the boron concentration data from the data management module 6, establishes a voxel prosthesis tissue model having a tissue type and a tissue boron concentration in combination with the medical image data, and simulates collision trajectories and energy distributions of nuclear particles in an internal 3D space through a Monte Carlo simulation program when the irradiated body S is irradiated by a neutron beam. Through sampling at different irradiation angles, a dose distribution of the 3D voxel prosthesis tissue model at the different irradiation angles and different irradiation time is simulated and calculated. In combination with the dose distribution as well as a dose index (such as a prescribed dose), and a dose limit (such as an average dose and a maximum dose), the treatment plan is generated. A neutron beam model established based on beam source data of the neutron irradiation module 3 is provided in the treatment planning module 5.

The treatment plan is stored to the data management module:
The treatment planning module 5 transmits the treatment plan to the data management module 6. The data management module 6 stores the treatment plan. A setup module 4 acquires the treatment plan from the data management module 6 and calculates a setup position corresponding to the treatment plan. In this step, the setup position is a preset setup position. The preset setup position is transmitted to the data management module 6.

A simulation placement module 8 acquires the preset setup position from the data management module 6, and drives a simulation mechanical arm to move, thereby driving a simulation placement table 82 to move the irradiated body S to the preset setup position. During movement, because of interference between the simulation placement table 82 or the simulation mechanical arm or the irradiated body S and the device, the irradiated body S cannot reach some preset setup position. If the irradiated body S cannot reach the preset setup position, the simulation placement module 8 transmits information that the preset setup position is unreachable to the data management module 6. The data management module 6 transmits the information that the preset setup position is unreachable to the treatment planning module 5. The treatment planning module 5 updates the treatment plan. This step is repeated, until the irradiated body S can reach the preset setup position corresponding to the treatment plan, thereby realizing an effect that a plurality of system devices cooperate to automatically adjust the treatment plan. In a more preferred implementation, if the irradiated body S cannot reach the preset setup position, the setup module 4 calculates setup adjusting information and transmits the setup adjusting information to the data management module 6. The data management module 6 transmits the setup adjusting information to the treatment planning module 5. The treatment planning module 5 updates the treatment plan based on the setup adjusting information, thereby automatically adjusting the treatment plan quickly and efficiently. Certainly, in other optional implementations, if the irradiated body S cannot reach the preset setup position, the treatment plan is directly updated by an operator, and a new treatment plan is transmitted to the data management module 6.

In the embodiment, whether the treatment plan is feasible is verified by the simulation placement module 8 in the preparation room 20. In other embodiments, this step can be performed by a placement module 7 in the irradiation room 30. The specific step refers to the above description, and is not repeated herein. It may be understood that the boron concentration may be a preset boron concentration, and may also be an actual boron concentration detected by the boron concentration acquisition module 2.

A setup motion parameter is acquired:
The setup module 4 acquires the treatment plan from the data management module, and performs calculation based on the treatment plan to obtain a setup position to be reached by the irradiated body S in irradiating treatment. Further, the setup module calculates the setup motion parameter according to the setup position, and transmits the setup motion parameter to the data management module 6.

Simulated setup is performed:
The simulation placement module 8 acquires the setup motion parameter from the data management module 6, and controls the simulation mechanical arm based on the setup motion parameter to move, thereby driving the simulation placement table 82 to move the irradiated body S to the setup position. The setup position in this step is a simulated setup position. Then, a position where an intersection point for three laser beams of a first positioning device 21 intersects with the irradiated body S is marked.

Treatment setup is performed:
The placement module 4 acquires the setup motion parameter from the data management module 6, and controls a drive portion according to the setup motion parameter to drive a mechanical arm 72, thereby driving the placement table 71 to move the irradiated body S to the setup position and generating setup in-place information. The placement module 4 transmits the setup in-place information to the data management module 6. The setup position in this step is a treatment setup position. Specifically:
Upon the simulated setup, while a relative position relationship between the irradiated body S and the simulation placement table 82 is unchanged, the simulation placement table 82 is separated from the simulation mechanical arm. The simulation placement table 82 tied with the irradiated body S is transported by a device such as a trolley to the irradiation room 20, and the simulation placement table 82 is provided on the mechanical arm 72. In the embodiment of the present disclosure, the placement table 71 in the irradiation room 20 is the simulation placement table 82 transported from the preparation room 30 to the irradiation room 20.

In the implementation, in the preparation room 30 and the irradiation room 20, a relative position relationship between the simulation mechanical arm and the first positioning device 21 as well as the simulation beam outlet 81 is completely the same as a relative position relationship between the mechanical arm 72 and the second positioning device 31 as well as the beam outlet 325. If the intersection point for the laser beams of the second positioning device 31 coincides with a marker of the irradiated body S, it is determined that the placement module 7 is set up in place, and the placement module 7 generates setup in-place information and transmits the setup in-place information to the data management module 6. If the intersection point for the laser beams of the second positioning device 31 does not coincide with the marker of the irradiated body S, it is determined that the placement module 7 is not set up in place, and the position of the placement table 71 is to be adjusted according to the marker, until the intersection point for the laser beams of the second positioning device 31 coincides with the marker of the irradiated body S.

In other implementations, the first positioning device 21 and the second positioning device 3 may also not be provided. The setup module 4 calculates a simulated setup motion parameter in the simulated setup. In combination with a difference between the relative position relationship between the simulation mechanism arm and the simulation beam outlet 81 and the relative position relationship between the mechanical arm 72 and the beam outlet 325 in the preparation room 30 and the irradiation room 20, the setup module calculates the setup motion parameter by compensation. When the mechanical arm 72 moves in place according to the setup motion parameter, the placement module 7 is set up in place by default.

Irradiation is performed:
An irradiation control module 9 acquires a setup in-place confirmation result from the data management module 6, performs other safety inspections upon determination of in-place setup, and starts up an associated device for neutron beam irradiating treatment after the other safety inspections meet requirements.

Further, during the neutron beam irradiating treatment on the irradiated body S, the boron is continuously supplied to the irradiated body S. When the boron concentration in the irradiated body S changes, a neutron dose in the nuclear reaction changes correspondingly. At this time, the irradiation time is adjusted according to an actual boron concentration in the irradiated body S. Specifically: The boron concentration acquisition module 2 acquires the boron concentration timely or periodically, and transmits a detected result to the data management module 6. The data management module 6 adjusts remaining irradiation time in combination with a resulting treatment plan and a present boron concentration, and transmits the remaining irradiation time to the data management module 6. The irradiation control module 9 acquires the remaining irradiation time from the data management module 6, controls operation time of the device according to the remaining irradiation time, and turns off the device when the remaining irradiation time becomes zero to stop the neutron beam irradiating treatment.

Further, the BNCT system further includes a display module configured to display, in the whole treatment process, the treatment plan at least including the irradiation time. The display module may be provided individually, and may also be integrated with the irradiation control module 7 or the data management module 6.

The BNCT system may further include a control room (not shown) and other spaces configured to assist the treatment. The operator controls the progress of the irradiating treatment in the control room.

In other implementations, the irradiation control module 7 may not be provided individually, but the irradiation control module 9 is integrated to the data management module 6. That is, the data management module 6 has functions of data storage, integration and system control.

In the embodiment, the setup module 4 calculates the setup motion parameter and transmits the setup motion parameter to the data management module 6. The placement module 7 and/or the simulation placement module 8 acquires the setup motion parameter from the data management module 6 to control a motion of each of the mechanical arm 72 and the simulation mechanical arm. In other embodiments, the setup module 4 can directly control the mechanical arm 72 and the simulation mechanical arm to move the irradiated body S to the treatment setup position and the simulated setup position. In other implementations, the preparation room 30 may not be provided, and the simulated setup is omitted accordingly. The placement module 4 acquires the setup motion parameter from the data management module 6, and performs setup in the irradiation room 20 according to the setup motion parameter. Upon in-place setup, the setup module 4 transmits the setup in-place information to the data management module 6. The irradiation control module 7 acquires the setup in-place confirmation result from the data management module 6, performs other safety inspections upon determination of the in-place setup, and starts up an associated device for neutron beam irradiating treatment after the other safety inspections meet requirements.

The preset setup position, the simulated setup position, and the treatment setup position each are the setup position obtained by the setup module 4 based on the treatment plan. For ease of identification, the setup position uses different names according to different steps for acquiring the setup position and different spaces. The preset setup position, the simulated setup position, and the treatment setup position are collectively called the setup position. Without a particular emphasis, the placement module may be understood as the placement module 7 or the simulation placement module 8.

According to the present disclosure, the BNCT system includes the data management module 6 configured to store information generated by at least one of the boron concentration acquisition module 2, the treatment planning module 5, the neutron beam irradiation module 3, the setup module 4, the placement module 7, and the simulation placement module 8. In work of the BNCT system, relevant data can be transmitted to the data management module 6 by at least one of remaining modules, and desired data can be acquired from the data management module 6 to perform corresponding calculation and/or control. Hence, the associated module can be turned off or works in a next round after completing the corresponding work. This prevents the resource waste and the radiation damage caused by unnecessary startup, and improves the operation efficiency of each device. For example, after acquiring the concentration and transmitting the detected data to the data management module 6, the boron concentration acquisition module 2 can acquire a boron concentration of another irradiated body S, thereby greatly improving the utilization rate of the device. In addition, the data management module 6 receives and stores the information data generated by at least one of the boron concentration acquisition module 2, the neutron beam irradiation module 3, the setup module 4, the treatment planning module 5, the placement module 7, and the irradiation control module 9, and/or perform information interaction with at least one of these modules. This simplifies the data processing procedure, improves the degree of automation of the BNCT system, and reduces the operation complexity.

Still further, in the work of the BNCT system, the boron concentration acquisition module 2 detects the boron concentration in the irradiated body S timely or periodically, and adjusts the irradiation time according to the detected boron concentration. This ensures that the actually implemented irradiating treatment is the same as the treatment plan.

In conclusion, the BNCT system in the present disclosure makes the treatment more automatic, improves the operation efficiency of each device, and ensures that the actually implemented irradiating treatment is the same as the treatment plan. Meanwhile, the present disclosure reduces the human error, and the radiation exposure time of the operator.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that, for a person of ordinary skill in the art, several variations and improvements can be made without departing from the concept of the present application, all of which fall within the protection scope of the present application. Therefore, the protection scope of the present disclosure should be subject to the protection scope defined by the appended claims.

## Claims

1. A boron neutron capture therapy (BNCT) system, comprising:
a boron concentration acquisition module configured to acquire blood boron concentration data of an irradiated body;
a neutron beam irradiation module configured to generate a neutron beam;
a treatment planning module configured to generate a treatment plan;
a setup module at least configured to calculate a setup position of the irradiated body;
a placement module configured to move the irradiated body to the setup position; and
a data management module configured to store information data generated by at least one of the boron concentration acquisition module, the neutron beam irradiation module, the treatment planning module, the setup module, and the placement module, and/or perform information interaction with at least one of the boron concentration acquisition module, the neutron beam irradiation module, the treatment planning module, the setup module, and the placement module.

2. The BNCT system according to claim 1, further comprising an image acquisition module configured to acquire medical image data of the irradiated body, wherein the treatment planning module is configured to generate the treatment plan based on the medical image data; and the treatment planning module is configured to perform information data interaction with the data management module and store the treatment plan to the data management module.

3. The BNCT system according to claim 1, wherein the setup module is configured to acquire the treatment plan from the data management module, and perform calculation based on the treatment plan to obtain a simulated setup position.

4. The BNCT system according to claim 3, wherein the setup module is configured to perform calculation based on the simulated setup position to obtain a setup motion parameter; and the placement module is configured to move the irradiated body based on the setup motion parameter to a treatment setup position.

5. The BNCT system according to claim 4, wherein after the irradiated body is moved to the treatment setup position, the placement module is configured to generate setup in-place information and transmit the setup in-place information to the data management module.

6. A working method of a boron neutron capture therapy (BNCT) system, comprising the following steps:
generating, by a treatment planning module, a treatment plan based on medical image data;
storing the treatment plan to a data management module;
acquiring, by a setup module, the treatment plan from the data management module, and calculating a setup position of an irradiated body based on the treatment plan;
moving, by a placement module, the irradiated body to the setup position; and
acquiring, by an irradiation control module, the treatment plan from the data management module, and controlling a neutron beam irradiation module based on the treatment plan to generate a neutron beam.

7. The working method according to claim 6, wherein the treatment planning module is configured to define a tissue boron concentration in the medical image data of the irradiated body, acquire a voxel prosthesis tissue model having a tissue type and the tissue boron concentration, and perform simulation based on the voxel prosthesis tissue model to generate the treatment plan.

8. The working method according to claim 6, wherein the setup module is configured to acquire the treatment plan from the data management module, acquire a preset setup position based on the treatment plan, evaluate whether the preset setup position meets a preset condition, update the treatment plan if the preset setup position does not meet the preset condition, and store an updated treatment plan to the data management module.

9. The working method according to claim 6, wherein the setup module is configured to acquire the treatment plan from the data management module, acquire a simulated setup position based on the treatment plan, and further perform calculation based on the simulated setup position to obtain a setup motion parameter; and the setup module is configured to control the placement module based on the setup motion parameter to move the irradiated body to a treatment setup position.

10. The working method according to claim 6, wherein the setup module is configured to acquire the treatment plan from the data management module, acquire a simulated setup position based on the treatment plan, perform calculation based on the simulated setup position to obtain a setup motion parameter, and store the setup motion parameter to the data management module; and the placement module is configured to acquire the setup motion parameter from the data management module, and move the irradiated body based on the setup motion parameter to a treatment setup position.
